# EUROPEAN PATENT APPLICATION

(11) **EP 1 233 271 A1**
(43) Date of publication of application: **21.08.2002**
(21) Application number: 01103792.6
(22) Date of filing: 16.02.2001
(51) Int. Cl.: G01N 33/68

(54) **Method for identifying compounds modulating the MAPK activity**

(71) Applicant: Huber, Lukas, 2103 Langenzersdorf (AT); Wunderlich, Winfried, 1210 Wien (AT); Fialka, Irene, 1060 Wien (AT)
(72) Inventor: Huber, Lukas, 2103 Langenzersdorf (AT); Wunderlich, Winfried, 1210 Wien (AT); Fialka, Irene, 1060 Wien (AT)

(57) **Abstract**

In a method for identifying compounds for the treatment of disorders mediated by deregulation of the MAPK pathway related to structural and/or functional alterations of the endosomal/lysosomal system, compounds are tested for their ability to inhibit, on late endosomes/lysosomes, the interaction of p14 with MP1 or the interaction of the p14/MP1 complex with components of the MAPK pathway.

SEQUENCE LISTING

<110> Huber, Lukas

<120> Method for identifying compounds modulating the MAPK
      activity

<130> HLA1

<140> EP 01103792.6
<141> 2001-02-16

<160> 3

<170> PatentIn Ver. 2.1


<210> 1
<211> 24
<212> PRT
<213> Mus musculus

<400> 1
Cys Val Ser Asp Arg Asp Gly Val Pro Val Ile Lys Val Ala Asn Asp
  1               5                  10                  15

Ser Ala Pro Glu His Ala Leu Arg
             20


<210> 2
<211> 10
<212> PRT
<213> Mus musculus

<400> 2
Lys Glu Thr Val Gly Phe Gly Met Leu Lys
  1               5                  10


<210> 3
<211> 17
<212> PRT
<213> Mus musculus

<400> 3
Lys Ala Gln Ala Leu Val Gln Tyr Leu Glu Glu Pro Leu Thr Gln Val
  1               5                  10                  15

Ala

## Description

The present invention relates to the therapy of diseases associated with the activation of the MAPK (Mitogen Activated Protein Kinase) / ERK (Extracellular Signal Regulated Kinase) signalling pathway.

The MAPK pathway is a ubiquitously expressed signalling module in vertebrate and invertebrate organisms, which governs the proliferation, differentiation and survival of cells (1,2). It has been the subject of numerous studies in the past years. The basic setup of this pathway is a cascade of three kinases, Raf->MEK->MAPK/ERK, that sequentially activate each other (1,3,4). Raf is thought to integrate the upstream input signals, while ERK is considered the effector end with an impressive roster of more than 50 substrates described to date.

There are several possibilities to explain how the MAPK pathway conveys specificity given all the pleiotropic biological responses it is involved in. First, there is some evidence that the amplitude, duration and kinetics of ERK signalling determine the biological outcome. For instance, a short transient activation is insufficient for induction of DNA synthesis, which requires a second wave of sustained ERK activity (5). A sustained high ERK activity arrests the cells in G1, because it triggers expression of the p21^{CIP} cell cycle inhibitor (6). These kinetic parameters are subject to modulation by associated proteins, feedback regulations within the MAPK pathway (7-9) and cross-regulation by other signalling pathways, especially the cAMP system (10-13). Second, the MAPK pathway may not be strictly linear as usually assumed, and there may be independent pathways branching off at the level of Raf and MEK. Third, all the components of the MAPK module undergo dynamic subcellular redistributions, which may determine the access to activators and substrates and hence specify which downstream targets are selected.

Signalling occurs in multiprotein complexes whose composition undergoes dynamic changes upon activation. These "signalosomes" contain activator, modulator and substrate proteins. There is evidence for differential subcellular redistribution of the components of this pathway in response to stimulation. The subcellular localisation determines interactions with upstream activators, modulators and downstream substrates, and hence plays a major role in the regulation of the activity of the pathway.

For a long time it was thought that the major impact of endocytosis on signaling is by downregulating the number of surface receptors, such as the EGF receptor one of the major singnal inputs into the MAPK signalling cascade (14). Using dominant negative dynamin in EGF activated cells, it became clear that the EGF receptor activates its targets Ras, Raf and MAPK/ERK kinase (MEK) at the plasma membrane. But endocytosis has to occur to activate the MAPK extracellular signal regulated kinase (ERK) (15). Of additional interest in this context is that members of this growth factor family, that differ in their intracellular fates, also differ in their signaling properties. ErbB1 for instance is routed for lysosomal degradation only when induced by EGF but recycles when binding TGFα. In contrast, ErbB3, whose ligands are the neuregulins, is always recycled (16). Another receptor for which membrane traffic has been shown to be important is PAR2, a member of the PAR family of G protein coupled receptors. Endocytosis of activated PAR2 is necessary for the activated receptor to meet its downstream effectors Raf and ERK on the endocytic compartment. By this, activated ERK is sequestered and kept away from entering the nucleus, thereby achieving substrate selectivity (17). These functional studies together with localization data detecting members of the MAPK module on endocytic structures (15, 18) emphasized the importance of endocytosis as regulator of signal transduction (19).

Within epithelial cells an additional level of complexity is added to the organizational skills of a cell. Apical-basal polarity separates the epithelial cell in two distinct domains. Correct sorting of receptors and downstream effectors is crucial for proper flow of information (21,22). Disturbance of this organization leads to pathophysiological consequences like increased autocrine stimulation of EGFR in cyst epithelia in polycystic kidney disease (22) and crypt epithelia of colon carcinoma (23) or ablation of an EGFR dependent vulva inducing signal in C.elegans (24, 25).

Another important concept emerging during the last years aims to explain how an activated enzyme selects the appropriate substrates by scaffolding (26, 27). Several proteins have been identified that influence signaling by routing certain partners of a cascade together in one complex. The cellular repertoire of such molecules spans from organizers of giant 'signalosomes' like inaD to simple trimeric complex builders like MP1. Their role in signal transduction is thought to enhance specificity and selectivity by bringing together components of a given pathway and separating them from other upstream activators as well as from downstream targets (anchoring scaffolds) or bringing the selected partners in close proximity (catalytic scaffolds) (28).

It was an object of the invention to further characterize the MAPK signalling pathway, in particular to identify key regulatory components and their function in this pathway. These components and underlying mechanisms provide novel targets for the therapeutic intervention in diseases where a deregulation of MAPK signalling is causally involved.

To solve the problem underlying the invention, in a first aspect, it was sought to obtain exact information about the localisation of the proteins and their interactions over a timecourse and correlate this with changes in activities. To better understand how a cell organizes spatio temporal patterns of signal transducing elements protein complexes were analyzed that are formed on and/or recruited to intracellular membranes upon signaling. The important question in this respect was, whether scaffolding occurs on intracellular membranes. This question was approached by analyzing the proteomes of endocytic organelles by a combination of subcellular fractionation, 2D-gel electrophoresis and microsequencing.

The present invention is based on the identification and characterization of a hitherto uncharacterized protein. In the following, this protein is termed "p14". p14 localizes to late endosomes/lysosomes where it interacts with the MAPK scaffold MP1 (40). Both proteins can be found in complex with elements of the MAPK module suggesting that the MAPK ERK1 is recruited to and activated on late endosomes/lysosomes or that these organelles are direct targets of this pathway.

The findings of the present invention have, for the first time, provided evidence for a link between the MAPK pathway and the late endosomal/lysosomal compartment.

Based on these findings, the present invention relates to a method for identifying compounds for the treatment of disorders mediated by deregulation of the MAPK pathway related to structural and/or functional alterations of the endosomal/lysosomal system , which is characterized in that compounds are tested for their ability to inhibit, on late endosomes/lysosomes, the interaction of p14 with MP1 or the interaction of the p14/MP1 complex with components of the MAPK pathway.

Examples for disorders where the compounds identified in the method of the invention may be beneficial are lysosomal storage/transport diseases, e.g. Cystic Fibrosis (acidification of endocytic organelles), Nieman-Pick-disease (lysosomal storage, lipid transport), or diseases that affect lysosomal secretion, e.g. Asthma (mast cell degranulation) or Chediak-Higashi syndrome. In addition also those neoplastic transformations where MAPK signalling is obviously deregulated may be included.

In a first embodiment of the invention, the method employs, as a first step, a cell-free assay.

The principle of such an assay is to inhibit the physical interaction of p14 and MP1; preferably the human proteins are used. Due to the high homology of murine p14 and MP1 with the human orthologues (e.g. murine and human p14 differ only in a single aminoacid), the murine orthologues or combinations of human and murine proteins can be used. Preferably, the recombinant proteins are used.

The biochemical tools for the assay are readily available. Both the p14 murine and human cDNAs are available from GenBank (murine: Acc: AA250088; human: Acc: NM_014017).

The proteins can be expressed by transfection and growth of suitable hosts according to standard protocols (in Examples 2 and 3, the expression of both proteins is described). Suitable hosts are higher eukaryotic cells like a mammalian cell or an insect cell, or a yeast or a bacterial cell, they are used according to routinely available expression methods.

Since the experiments of the invention show that the p14/MP1 interaction is not disturbed by large tags such as GST, GFP on either of the binding partners, proteins that carry such tags from the precedent purification may also be used.

The assay may be conducted by means of an in vitro interaction system (pulldown assay of recombinant protein or of ectopically expressed protein in mammalian cells) as described in Example 3.

The first step of the screening method is based on the disruption of a p14/MP1 complex and can be carried out in the following manner:
1) immobilizing one of the interaction partners p14 or MP1 on a matrix (plate or beads) according to standard methods (e.g. via commercially available tags like GST, Histidine, Maltose Binding Protein (MBP), Thioredoxin; unspecific binding to plastic surfaces, e.g. multiwell plates; Biochips)
2) labeling the other interaction partner with a detectable marker. Preferred markers are those producing a signal that can be easily measured in a high throughput screen. Examples for markers useful in the present invention are known in the art, they are selected from radioactive labels, e.g. ¹²⁵Iodine, commercially available fluorescent markers for labelling proteins or peptides. e.g. Europium or the Green Fluorescent Protein (GFP), enzymes, e.g. luciferase, alkaline phosphatase etc.
3) performing the binding reaction between MP1 and p14 in the presence or absence of the test compound (potentially inhibitory peptides or small molecular weight compounds) or incubating the p14/MP1 complex in the presence or absence of the test compound.
4) determining, preferably in multiwell format, signal quenching (comparison of the signal intensity indicative of the efficiency of the p14/MP1 complex formation/complex stability in the presence and absence of the test compound).

In a variation of this assay, compounds are tested, in. the first step, for their ability to interfere with the interaction of the p14/MP1 complex with members of the MAPK pathway, e.g. MEK1 and/or ERK1 and other proteins that bind to MEK1 or ERK1, either directly or indirectly. Examples are KSR, Raf, RKIP, Hsp90, 14-3-3, etc.

In this embodiment, the p14/MP1 complex can be obtained either by in vitro assembly of the purified components, which have been obtained as described above, or by stoichiometric co-expression of p14 and MP1 in a single host cell. The expression vehicle may be composed of a combination of commercially available standard expression plasmids carrying the cDNA of either partner protein.

This variation of the assay can be carried out according to the same principle as described above, with the difference that the p14/MP1 complex represents one of the interaction partners. Thus, either the complex or one of the above-described directly or indirectly interacting proteins may be immobilized and the other is carrying the label.

In another embodiment of the invention, compounds are screened, in the first step, for their ability to disrupt the p14/MP1 interaction in a cellular assay.

In this embodiment, constructs used in the two hybrid screen may be directly used, as desribed in Example 3 where MP1 was identified as binding partner of p14. These constructs can be used in a modified version of the yeast two hybrid screen by using a different reporter construct.

This so-called "counterselection yeast two-hybrid assay" differs from the originally described interaction screen of Example 3 in the following way: It utilizes a lethal or growth inhibitory reporter construct whose expression depends on the interaction of p14 and MP1. To screen for inhibition of the p14/MP1 interaction either a peptide library is transfected into yeast cells or chemical libraries with small molecular weight compounds are employed.

This system can, in a further embodiment, also be adapted for mammalian cells with the advantage of screening at the same time for cell membrane permeable inhibitors.

Examples for genes suitable for a reporter construct in a counterselection two hybrid screen are RAP1 (42), HIV-1 integrase (43); actin (44); CYH2 (45).

In the next step of the screening assay, the test compounds identified in step 1 are tested for their ability to restore disorders related to endosomal/lysosomal phenotypes, e.g. defects in storage, transport and/or acidification. To this end, cell lines, in particular human cell lines from patients with such diseases (examples of which have been described above), can be used; such cell lines are available in the art, e.g. from cell culture collections like ATCC. Cells are treated with the candidate compounds and endosomal/lysosomal compartments analyzed by standard cell biology techniques (e.g. immunofluorescence microscopy with markers specific for the compartment, as described in Example 2) or biochemically (e.g. organelle proteomics, subcellular fractionation as described in Example 1).

In addition, the MAPK pathway is deregulated in more than 30% of human cancers, including prevalent and aggressive cancers such as lung, ovarian, kidney, colon (>50%) and pancreas (>80%) cancer. Furthermore, recent studies show its involvement in lung diseases, infectious diseases, cardiovascular conditions like cardiac hypertrophy and stroke. Compounds identified in the method of the invention have the potential to be of therapeutic value for the treatment of such diseases, where the above-described link to late endosomes/lysosomes becomes evident.

To extend the applicabiliy of the compounds that have proven to have an effect on endosomal/lysosomal phenotype, for cancer therapy, they may be additionally tested for inhibition of tumor cell proliferation. To this end, primary human tumor cells, e.g. cell lines derived from epithelial tumors, are incubated with the compound identified in the primary screen and the inhibition of tumor cell proliferation is tested by conventional methods, e.g. bromo-desoxy-uridine or ³H incorporation.

Compounds that exhibit an anti-proliferative effect in these assays may be further tested in tumor animal models and used for the therapy of tumors. The findings of the invention open new avenues for therapeutic intervention, since inhibitors that act on the p14/MP1 complex thereby affecting MAPK signalling could inhibit tumor growth and improve the health status in human tumor patients.

Compounds that have been identified in the above-described screening methods may subsequently be subjected to a secondary screen to exclude compounds having non-specific cytotoxic effects. A suitable, nonradioactive cytotoxicity assay that may be employed in the secondary screen is the LDH (lactate dehydrogenase)release assay. LDH release assays are commercially available (e.g. Cytotox 96, Promega, Madison, Wisconsin, USA). Alternatively, a propidium iodide cytotoxicity assay may be employed.

Toxicity and therapeutic efficacy of a compound identified as a drug candidate can be determined by standard pharmaceutical procedures, which include conducting cell culture and animal experiments to determine the IC₅₀, LD₅0, the ED₅₀. The data obtained are used for determining the human dose range, which will also depend on the dosage form (tablets, capsules, aerosol sprays, ampules, etc.) and the administration route (oral, buccal, nasal, paterental or rectal). A pharmaceutical composition containing the compound as the active ingredient can be formulated in conventional manner using or more physiologically active carriers and excipients. Methods for making such formulations can be found in manuals, e.g. "Remington Pharmaceutical Sciences"

The findings of the present invention provide the basis to establish *in vitro* and *in vivo* systems for the analysis of how p14 and MP1 interact with each other and how this interaction on late endosomes/lysosomes influences the generation of specific signals and biological responses, e.g. through MEK1 and ERK1. For instance, understanding how the MAPK pathway conveys differentiation signals versus oncogenic signals may enable to selectively block the harmful signal while preserving the beneficial function. For this purpose the topological organisation of the MAPK pathway, its interaction with other proteins, and how these parameters relate to specific biological activities connected to structure and/or function of late endosomes/lysosomes are investigated by applying genetic model systems. Suitable model systems comprise "gain of function" and/or "loss of function" systems in various organisms. In these systems, both interaction partners p14 and MP1 are targets for gene deletions and overexpression in genetically modified organisms, e.g.

Drosophila melanogaster, Dictyostelium Discoideum and mice.

The goal of these studies is the identification of target proteins and, in particular the (patho)physiological processes of late endosomes/lysosomes that are connected to the MAPK pathways and are directly or indirectly, e.g. by activation, regulated by the interaction of p14 and MP1 on this compartment.

This is the prerequisite for the development of more specific drugs that selectively inhibit a defined subset of the functions of the MAPK cascade, for instance by restraining them on late endosomes/lysosomes or by preventing specific interactions at this localization.

Such specific inhibitors can be expected to be more efficient and patient-friendly than the treatments currently available. They would n'ot only contribute to improve the quality of life of individual patients, but also to relieve the economic strain that these diseases impose on our society.

### Brief description of the Figures

Figure 1. Subcellular localization of p14
Figure 2. Interaction between p14 and MP1 in vivo I - Co-Immunoprecipitation and Co-Sedimentation
Figure 3. Interaction between p14 and MP1 in vitro

In the following Examples, if not otherwise stated, the following Materials and Methods were used:
Cells and Tissue Culture
EpH4 murine mammary epithelial cells (46), Caco-2 cells and HeLa cells were grown in high glucose DMEM supplemented with 10 mM HEPES (pH 7.3), 50 IU/ml penicillin, 50 µg/ml streptomycin, and 5% FCS at 37°C, in 5 % CO₂ and 98 % humidity. Media and reagents for tissue culture were purchased from Gibco BRL (Life Technologies) and fetal calf serum (FCS) was obtained from BioWhittaker (Boehringer, Ingelheim).

### Antibodies

Polyclonal anti-p14 antiserum was raised against a GST fusion protein of p14. Anti-MP1 antibodies were raised against the peptide Kp532 (CVSDRDGVPVIKVANDSAPEHALR, aa 24-46, mouse MP1, Acc. No. AF082526, SEQ ID NO:1), respectively, and the latter affinity-purified on Affi-Gel matrix (BioRad) according to the manufacturer's instructions. Polyclonal antibodies recognizing the myc-epitope were obtained from Gramsch Laboratories (Schwabhausen, Germany). Antibodies specific for double phosphorylated ERK1/2 or MEK1/2 were purchased from New England BioLabs. Anti-His₆ and anti-Xpress antibodies were from Invitrogen and the anti-CD107a (LAMP-1) antibody was obtained from Pharmingen. Polyclonal anti-GST antibodies were generated in the lab. Alexa 488™, Alexa 568™, Cy3™- and Texas Red-labeled secondary antibodies were obtained from Molecular Probes, Amersham Life Science and Jackson ImmunoResearch Laboratories, respectively. LysoTracker™ Red DND-99 and EGF-Rhodamine were from Molecular Probes. Anti-EEA1 (49) and anti-Rabll (37) antibodies were generous gifts from Dr. Marino Zerial (EMBL, Heidelberg).

### Cell Homogenization and Membrane Preparation

EpH4 cells were homogenized and post nuclear supernatant (PNS) was prepared in homogenization buffer (HB: 250 mM sucrose, 3 mM imidazole, pH 7.4, 1 mM EDTA containing a cocktail of protease inhibitors: 10 µg/ml aprotinin, 1 µg/ml pepstatin, 10 µg/ml leupeptin and 1 mM Pefabloc SC, Boehringer-Mannheim) as outlined previously (20; 47; 31). Continuous sucrose gradients were used to separate different membrane compartments as described (20). Peripheral membrane proteins were separated from integral membrane proteins by extraction with 0.1 M Na₂CO₃, pH 11.0 (33).

### Two-dimensional Gel Electrophoresis (2D-GE) and Peptide Sequencing

2D-GE and microsequencing of protein spots were performed as described in detail in (29; 30; 31).

### Indirect Immunofluorescence

EpH4, HeLa and Caco-2 cells were fixed with 4% PFA (Paraformaldehyde) in CB (cytoskeleton buffer: 10 mM PIPES pH 6.8, 150 mM NaCl, 5 mM EGTA, 5 mM glucose, 5 mM MgCl₂) for at least 30 min, quenched by three washes with washing buffer (CB, 50 mM NH₄Cl) and permeabilized with CB supplemented with 0.3-0.5% TX-100. Specimen were processed for indirect immunofluorescence, mounted in Mowiol (Hoechst) and finally viewed using an Axioplan2 microscope (Zeiss). For confocal microscopy samples were mounted in 50% glycerol, 4% n-propyl gallate (Sigma) in CB. Confocal images were obtained with a Leica TCS NT confocal microscope (Leica, Heidelberg, Germany) and processed using the Imaris and Co-localization software packages (Bitplane AG, Zurich, Switzerland) after deconvolution using measured point-spread functions with the Huygens-software (Scientific Volume Imaging, Hilversum, Netherlands).

### Proteinase K Treatment of PNS

Equal volumes of PNS from EpH4 cells (~ 0.5 µg/µl protein) were treated with increasing concentrations of proteinase K (0.01-10 µg/ml, Gibco) for 20 min at room temperature. The reaction was stopped by addition of 100 mM PMSF. Undigested membrane material was pelleted at 100,000 xg and proteins analyzed by immunoblots.

### Two-Hybrid Screen

A two-hybrid screen was performed using the Matchmaker Gal4 Two-Hybrid System 2 (Clontech) following the manufacturer's screening protocol. Bait constructs were generated by PCR from the original clone obtained from the UK HGMP Resource Centre (I.M.A.G.E. Consortium CloneID 681056) (Lennon et al., 1996) using primers introducing an EcoRI site N-terminally and a PstI site C-terminally of the respective fragments and subsequent cloning of these fragments into pAS2-1 (Clontech). The resulting chimeric proteins consisted of the Gal4 DNA binding domain fused in frame to the full length protein, an N-terminal fragment of p14 (aa 1-48) or two different C-terminal fragments (C1: aa 43-125; C2: aa 80-125). After titration of the appropriate 3-amino-1,2,4-triazole (3-AT) concentration to inhibit background His3 activity, the different bait constructs were introduced into yeast strain HF7c, tested for autonomous activation and subsequently screened for interacting polypeptides using a mouse embryo MATCHMAKER cDNA library cloned into pACT2 (Clontech). The pAS2-1 C2 construct showed auto-activation resistant to 3-AT and, thus, was not used for further screening.

### Constructs and Transfection

Tagged versions of p14 and MP1 containing a triple myc-tag at the N-termini of the proteins were constructed by PCR using primers introducing appropriate restriction sites (p14) or by direct cloning from one of the positive pACT2 clones (MP1) into a BSK vector containing three myc sequences preceded by a Kozak sequence (48) that was constructed in our laboratory. The coding sequences of the resulting chimeric proteins (myc₃-p14 and myc₃-MP1) were cloned into expression vectors pREP10 (Invitrogen), resulting in sense or antisense myc₃-p14 constructs, and pUB6/V5-His (Invitrogen; out of frame of the C-terminal V5-His tag), respectively. CAAX-tagged p14 was constructed by introducing a linker sequence encoding the last 21 aa of human K-ras (41) at the C-terminus of the p14 cDNA replacing the STOP codon. Both bona fide p14 and p14-CAAX were cloned in frame with the His₆/Xpress-tag into pEF4/HisC (Invitrogen) to give rise to N-terminally tagged X-p14 and X-p14-CAAX expression vectors. EGFP-p14 was constructed by cloning the coding sequence of p14 into pEGFP-C1 (Clontech). Cells were transfected with the different constructs by use of Lipofectamin Plus (Gibco) following the manufacturer's suggestions and eventually selected accordingly for stable transfectants.

Recombinant proteins were constructed in pGEX6P3 (Pharmacia) or pET28 (Novagen).

### Immunoprecipitation

Transfected cells were scraped in PBS and lysed by a combination of a quick freeze-thaw cycle and sonication. After centrifugation at 1,600xg the supernatant was diluted in IP-buffer (10 mM HEPES, pH 7.4, 137 mM NaCl, 4.7 mM KCl, 0.65 mM MgSO₄, 1.2 mM CaCl₂, 1% TX-100, 2 mM NaF, 20 mM β-glycerophosphate and protease inhibitors as for cell homogenization). After pre-clearing with UltraLink™-immobilized Protein A (PIERCE), the resulting supernatant was subjected to immunoprecipitation using pre-immuneserum or polyclonal anti-myc antibodies and UltraLink™-immobilized Protein A, or Protein A alone as additional negative control. Following three washes with IP-buffer, the samples were boiled in loading buffer and resolved on SDS-PAGE.

### Glycerol Density Gradients

Total membranes from EpH4 were enriched by centrifugation of PNS at 100,000 xg, resuspended in extraction buffer (20 mM HEPES/KOH, pH 7.0, 100 mM KCl, 1 mM DTT, 1% TX-100, 2 mM NaF, 20 mM β-glycerophosphate, and cocktail of protease inhibitors as above) and extracted on ice for 30 min. The insoluble material was pelleted at 17,000 xg and the resulting supernatant (containing p14 and MP1) loaded on top of a continuous glycerol gradient (5-35% in extraction buffer). Gradients were centrifuged at 270,000 xg over night in a SW41 rotor (Beckman). Then, 600 µl fractions were collected, proteins precipitated and analyzed by 12.5% SDS-PAGE and immunoblots. For gradient calibration we used a protein mix (1 µg/µl BSA, 4.5 S; 2 µg/µl aldolase, 7.3 S; 2 µg/µl catalase, 10.0 S; and 1 µg/µl thyroglobulin, 19.5 S, in HB) the distribution of which was detected by Coomassie stain after SDS-PAGE.

### In vitro Pull-Down Assay

Bacterial lysates containing recombinant proteins were prepared by sonication in PBS. GST and GST-fusion proteins were bound to GSH-sepharose (Pharmacia), washed with PBS, and incubated with lysates containing His₆-tagged recombinant proteins for 20 min at room temperature. Subsequently, the sepharose-bound proteins were washed, resuspended in sample buffer and analyzed by SDS-PAGE and immunoblots. Because of the reported promiscuity in the *in vitro* interaction of MP1 with the different isoforms of the kinases (40) we used a GST-ERK2 construct that was obtained from M. J. Weber.

### Electron microscopy

Caco-2 cells expressing EGFP-p14 were fixed in 4% paraformaldehyde/0.1% glutaraldehyde in 0.1M phosphate buffer, pH 7.35, for 1h at RT. They were then washed with 0.2M phosphate buffer, scraped from the culture dish and pelleted in a microfuge. The cells were then resuspended in warm gelatin (10% in phosphate buffer) and repelleted at maximum speed in the microfuge. After cooling, the gelatin-embedded cells were infiltrated with PVP-sucrose overnight at 4°C and then processed for frozen sectioning as described (50). Ultrathin frozen sections (60-80nm) were labeled, stained, and viewed (Jeol 1010, Centre for Microscopy and Microanalysis) according to published techniques (51).

### Example 1

To investigate the protein composition of the endocytic compartment techniques were applied that have already been successfully used for the analysis of membrane associated organelle proteins within the mammary epithelial cell line EpH4 (29-32). First sucrose density gradient centrifugation of post nuclear supernatants (PNS) was performed to separate organelles derived from early and late endocytic compartments, respectively. Gradient enriched organelle fractions were then applied to high resoltution 2D-GE to obtain a protein pattern allowing the identification of proteins specifically enriched in late endosomal/lysosomal fractions. Since a lot of proteins described in regulating endocytic transport (e.g. Rab proteins) or scaffolding signal transducing modules (e.g. Ste5p) are peripherally associated with membranes, the fractions were further characterized by carbonate extraction at high pH (33, 31).

For this invention a particular protein spot with an experimental isoelectric point (pI) of about 5.0 and a molecular weight of about 14 kD is described in more detail. It was abundant and enriched in gradient purified organelle fractions containing mainly late endosomes and lysosomes when compared to PNS or early endosome containing fractions. In addition this protein was exclusively associated with membrane fractions of that density and never detected in the cytoplasmic fractions.

The spot was excised from a preparative gel (31), digested and microsequenced by Edman degradation. Two peptide sequences were obtained: KETVGFGMLK (SEQ ID NO:2) and KAQALVQYLEEPLTQVA (SEQ ID NO:3). These sequences were found to be part of open reading frames from a number of different EST (expressed sequence tag) clones, originating from a variety of different species, organs, developmental stages and cell lines. Comparison of the primary polypeptide sequences of different species revealed very high sequence conservation within multicellular organisms. However, there was no sequence with significant homology detectable in the yeast genome. Comparison with databases of known proteins did not reveal homology to any other existing protein or protein domain. The calculated pI (5.3) and molecular weight (13,480 D) of the mouse protein, now termed p14, corresponded well with the experimental data obtained from the 2D gels. On the transcriptional level a single RNA of 700bp was detectable in EpH4 cells. Ubiquitous expression could be demonstrated by using a multiple tissue northern blot from CLONTECH.

In a secondary analysis the membrane topology of p14 was investigated. The protein displayed features of a peripheral membrane protein since it was partially extractable by carbonate at high pH. Furthermore, PNS was prepared under conditions that maintain the integrity of vesicles and subjected to proteinase K digestion. Proteinase K is not membrane permeable and, thus, digests at limited concentrations only proteins accessible at the cytoplasmic face of membranes. Under these conditions p14 was much more sensitive to digestion than known luminal marker proteins, e.g. calreticulin, (34), or even proteins on the cytoplasmic side, e.g. Rab5, (35). Calreticulin was chosen since it represented a well-characterized lumenal marker protein of the ER, the most abundant vesicle fraction in PNS. Taken together these results suggested a peripheral association of p14 with the cytoplasmic face of membranes.

### Example 2

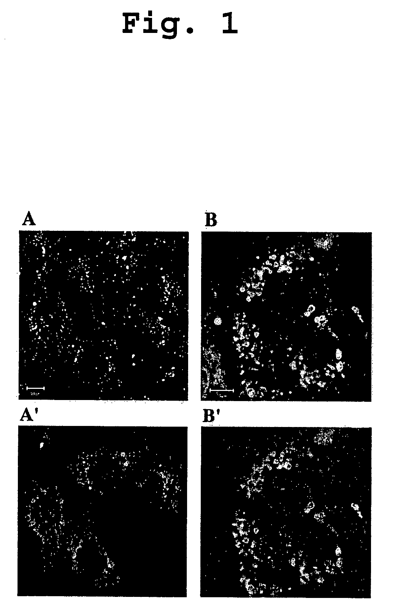

To confirm the subcellular localization of p14, an EGFP-tagged version of p14 was expressed in EpH4 cells and the cells incubated with LysoTracker™ Red DND-99, an acidotropic probe. Live microscopy revealed vesicular structures surrounded by the green fluorescence of EGFP-p14 and filled with the red fluorescence of the lysotracker (Fig. 1 A, A'), indicating a localization of p14 on acidic organelles such as late endosomes and lysosomes. To exclude mistargeting mediated by the EGFP-tag or by overexpression the analysis was extended with differently tagged versions of p14 in two other cell types (Caco-2 and HeLa). Another epithelial cell line, Caco-2 cells, stably expressing EGFP-p14 was used for co-immunofluorescence with LAMP-1, a transmembrane glycoprotein localizing mainly to late endosomes/lysosomes. In these cells, LAMP-1 showed a complete overlap with the exogenously expressed p14 (Fig. 1B, B'). This result was further confirmed by exploiting the well studied intracellular trafficking of the EGF receptor (EGFR) and its ligand. After binding to the EGFR at the cell surface, EGF gets internalized together with its receptor and is transported to the early then to the late endocytic compartment and finally destined to degradation in lysosomes (36). EpH4 cells transiently transfected with EGFP-p14 were allowed to internalize EGF-rhodamine for various time and were then observed live under a microscope. EGF, initially separated from the green fluorescence was subsequently filling the compartment labeled with EGFP confirming the association of p14 with late endosomes/lysosomes in living cells. In addition Hela cells stably transfected with EGFP-p14 were were analyzed in co-immunolocalization experiments with early endocytic marker proteins. Neither EEA1, involved in vesicular transport from the plasma membrane to the early endosomes (49), nor Rab11 which regulates recycling through the pericentriolar recycling endosome (37) showed any significant co-immunolocalization with p14.

Immunoelectron microscopy confirmed and extended these observations. The majority of the labeling for the EGFP-tagged protein was associated with large electron-lucent structures with internal vesicular or lamellar membranes that were labeled with antibodies to the late endosomal marker, LBPA. As shown previously (38) the LBPA antibody labeled the internal membranes of the late endosomes. In contrast, EGFP-p14 was predominantly although not exclusively associated with the cytoplasmic surface of the late endosomes. In addition, EGFP-p14 labeling was associated with putative lysosomal structures with an electron dense core and negligible LBPA labeling. Smaller spherical LBPA-negative multivesicular endosomal structures, morphologically identifiable as endosome carrier vesicles (39), also showed low but specific labeling with the anti-GFP antibody. Taken together, this novel peripheral membrane protein localizes to the cytoplasmic face of late endosomal and lysosomal compartments in a number of different cell types and species.

Figure 1 shows the subcellular localization of p14:
EpH4 cells, transiently transfected with EGFP-p14 (A'), were treated with lysotracker (A) Living cells were observed under a confocal microscope.
Caco-2 cells, stably transfected with EGFP-p14 (B'), were fixed and co-immunolocalization with LAMP-1 was performed (B). Sizebars are 10 µm.

### Example 3

In order to investigate the biological function of p14 we attempted to identify potential interaction partners of p14. Therefore, we performed two-hybrid screens using different parts of the p14 coding sequence (full length, an N-terminal and a C-terminal fragment) as bait fused to the Gal4 DNA binding domain. With each construct we screened 1.2-5.6 x 10⁵ independent clones of a mouse embryonic library. Among positive clones, several contained the complete coding region of MP1 (MEK Partner 1), a protein recently identified as a scaffold protein of the MAPK pathway (40). We obtained interacting MP1 clones with all three different bait constructs (see Table 1), suggesting that different parts of the relatively small p14 protein were involved in the interaction with MP1. Alternatively, the small overlap of the N- and C-terminal constructs may play a central role in the interaction with MP1.

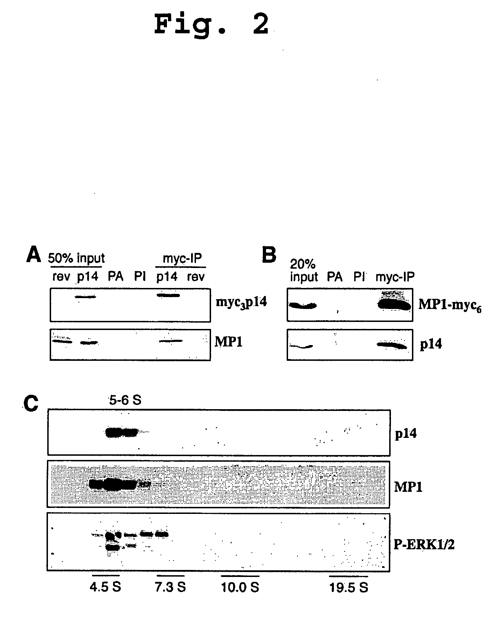

The interaction between p14 and MP1 was further confirmed with a variety of methods. First, the direct interaction between these two proteins was tested in a GST pull-down assay. GST-tagged p14 was able to directly interact with His₆-T7-MP1 (Fig. 2, lane 2). The specificity of this interaction was shown by the failure of His₆-T7-tagged MP1 to interact with GST alone (Fig. 2, lanes 3,4) and by the ability of excess His₆-tagged p14 to abrogate the GST-p14/His₆-T7-MP1 interaction (Fig. 2, lane 1). In addition His₆-tagged p14 weakly bound to GST-p14 (Fig. 2, lane 1) raising the possibility of a self-association.

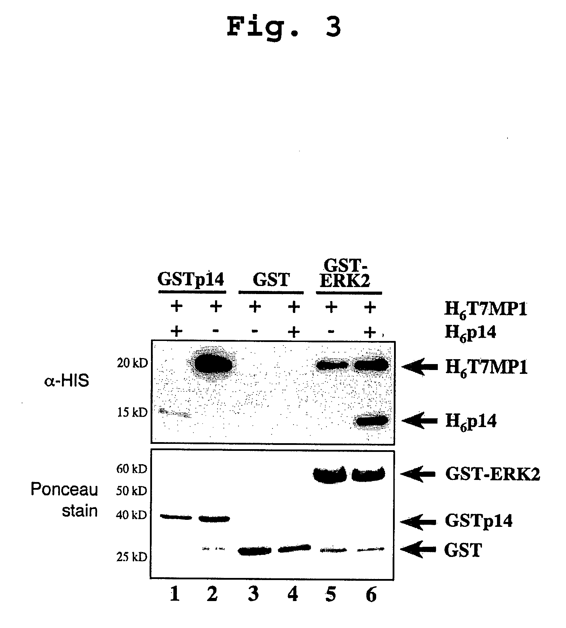

The direct interaction in the yeast two-hybrid assay and in pull-down assays in vitro demonstrated the ability of the two proteins to form a complex. Next we tested if this complex could also form in vivo. For this purpose we constructed HeLa cells expressing myc-tagged versions of p14 or MP1 and performed immunoprecipitations with anti-myc antibodies. In these experiments endogenous MP1 was detectable in immunoprecipitations of myc-p14 and endogenous p14 in immunoprecipitations of myc-MP1 (Fig. 3A) demonstrating an in vivo interaction of p14 and MP1. To further analyze a possible complex of p14/MP1 proteins we separated proteins and protein complexes after detergent extraction of a 100.000xg PNS pellet from EpH4 cells on continuous glycerol gradients. Herein endogenous p14 and MP1 co-fractionated with a similar sedimentation coefficient of around 5-6S (Fig. 3B).

Further support for a complex formation between p14 and MP1 in vivo came from co-localization experiments. In HeLa cells overexpressing Xpress-tagged p14 and myc-tagged MP1, both proteins localized to the same perinuclear, LAMP-1 positive compartment. In addition, fusing Xpress-tagged p14 to the hypervariable domain and CAAX-motif of K-Ras (41), leading to plasma membrane localization of p14, resulted in efficient recruitment of co-expressed MP1 to the same sites.

MP1 was identified as a complex partner for MEK1 and ERK1 in vivo as well as ERK2 in vitro (40). This prompted us to try to reconstitute in vitro complexes and to analyze complexes from cells for all of these components.

His₆-T7-MP1 efficiently bound to GST-ERK2 (Fig. 2, lane 5) and this interaction was not abrogated by excess His-tagged p14. Instead p14 bound to GST-ERK2 in addition to MP1 (Fig. 2, lane 6).

Interestingly, using untransfected cells we found double phosphorylated ERK1/2 co-sedimenting with p14 and MP1 in glycerol gradients but not activated MEK1/2.

Taken together the late endosomal/lysosomal peripheral membrane protein p14 specifically interacted with MP1 and members of the MAPK cascade.

The results are shown in Figure 2:
(A) Extracts from HeLa cells stably transfected with myc₃-p14 or a reverse myc₃-p14 construct as control were subjected to immunoprecipitation using polyclonal anti-myc antibodies. 50% of the extracts from the reverse control (rev) or myc₃-p14 expressing cells (p14) were analyzed as input controls. Immobilized Protein A alone (PA) or with pre-immune serum (PI) were used to control for unspecific binding. Co-immunoprecipitated MP1 was analyzed by immunoblotting.
(B) Extracts from HeLa cells stably expressing myc₃-MP1 were analyzed for co-immunoprecipitation of p14 as in panel A. 20% of the extract was loaded as input control.
(C) Total membranes of EpH4 cells were enriched by centrifugation at 100,000 xg. Extracted proteins were loaded onto a 5-35% glycerol gradient and collected fractions analyzed by immunoblotting. S-values of a mix of known proteins are indicated.

Figure 3 shows the interaction between p14 and MP1 in vitro

Recombinant GST-tagged p14 (lane 1,2), GST (lane 3,4) or GST-ERK2 (lane 5,6) were incubated with recombinant His₆-T7-tagged MP1 alone (lane 2, 3, 5) or with additional His₆-tagged p14 (lane 1, 4, 6). Amounts of GST proteins are illustrated in the lower panel by Ponceau staining.

**Table 1.**

| Summary of Two-Hybrid Screen for p14 Interaction Partners | | | | |
|---|---|---|---|---|
| Bait | Screened clones | Pos. clones | Typ. False Pos. | MP1 |
| Gal4-wt | 1.2 x 10⁵ | 24 | 2 | 15 |
| Gal4-N | 4.8 x 10⁵ | 5 | 0 | 1 |
| Gal4-C1 | 5.6 x 10⁵ | 5 | 3 | 1 |

### REFERENCES

1. Brunet, A. and Pouyssegur, J. Mammalian MAP kinase modules: how to transduce specific signals. (1997) *Essays. Biochem.* 32:1-16, 1-16
2. Lewis, T. S., Shapiro, P. S., and Ahn, N. G. Signal transduction through MAP kinase cascades. (1998) *Adv. Cancer Res.* 74:49-139, 49-139
3. Morrison, D. K. and Cutler, R. E. The complexity of Raf-1 regulation.(1997) *Curr. Opin. Cell Biol.* 9, 174-179
4. Marais, R. and Marshall, C. J. Control of the ERK MAP kinase cascade by Ras and Raf.(1996) *Cancer Surv.* 27:101-25, 101-125
5. Vouret-Craviari, V., Van Obberghen-Schilling, E., Scimeca, J. C., Van Obberghen, E., and Pouyssegur, J. Differential activation of p44mapk (ERK1) by alphathrombin and thrombin-receptor peptide agonist.(1993) *Biochem. J.* 289, 209-214
6. Lloyd, A. C., Obermueller, F., Staddon, S., Barth, C. F., McMahon, M., and Land, H. Cooperating oncogenes converge to regulate cyclin/cdk complexes.(1997) *Genes Dev.* 11, 663-677
7. Brondello, J. M., Brunet, A., Pouyssegur, J., and McKenzie, F. R. The dual specificity mitogen-activated protein kinase phosphatase-1 and -2 are induced by the p42/p44MAPK cascade.(1997) *J. Biol. Chem.* 272, 1368-1376
8. Brunet, A., Pages, G., and Pouyssegur, J. Growth factor-stimulated MAP kinase induces rapid retrophosphorylation and inhibition of MAP kinase kinase (MEK1).(1994) *FEBS Lett.* 346, 299-303
9. Zimmermann, S., Rommel, C., Ziogas, A., Lovric, J., Moelling, K., and Radziwill, G. MEK1 mediates a positive feedback on Raf-1 activity independently of Ras and Src.(1997) *Oncogene* 15, 1503-1511
10. Hoffmann, R., Baillie, G. S., MacKenzie, S. J., Yarwood, S. J., and Houslay, M. D. The MAP kinase ERK2 inhibits the cyclic AMP-specific phosphodiesterase HSPDE4D3 by phosphorylating it at Ser579.(1999) *EMBO J.* 18, 893-903
11. Mischak, H., Seitz, T., Janosch, P., Eulitz, M., Steen, H., Schellerer, M., Philipp, A., and Kolch, W. Negative regulation of Raf-1 by phosphorylation of serine 621.(1996) *Mol Cell Biol* 16, 5409-5418
12. Wu, J., Dent, P., Jelinek, T., Wolfman, A., Weber, M. J., and Sturgill, T. W. Inhibition of the EGF-activated MAP kinase signaling pathway by adenosine 3',5'-monophosphate [see comments].(1993) *Science* 262, 1065-1069
13. Häfner, S., Adler, H. S., Mischak, H., Janosch, P., Heidecker, G., Wolfman, A., Pippig, S., Lohse, M., Ueffing, M., and Kolch, W. Mechanism of inhibition of Raf-1 by protein kinase A.(1994) *Mol. Cell. Biol.* 14, 6696-6703
14. Di Fiore, P.P., and G.N. Gill. 1999. Endocytosis and mitogenic signaling. *Curr Opin Cell Biol.* 11:483-488.
15. Kranenburg, O., I. Verlaan, and W.H. Moolenaar. 1999. Dynamin is required for the activation of mitogen-activated protein (MAP) kinase by MAP kinase kinase. *J Biol Chem.* 274:35301-35304.
16. Baulida, J., M.H. Kraus, M. Alimandi, P.P. Di Fiore, and G. Carpenter. 1996. All ErbB receptors other than the epidermal growth factor receptor are endocytosis impaired. *J Biol Chem.* 271:5251-5257.
17. DeFea, K.A., J. Zalevsky, M.S. Thoma, O. Dery, R.D. Mullins, and N.W. Bunnett. 2000. beta-arrestin-dependent endocytosis of proteinase-activated receptor 2 is required for intracellular targeting of activated ERK1/2. *J Cell Biol.* 148:1267-1281.
18. Pol, A., M. Calvo, and C. Enrich. 1998. Isolated endosomes from quiescent rat liver contain the signal transduction machinery. Differential distribution of activated Raf-1 and Mek in the endocytic compartment. *FEBS Lett.* 441:34-38.
19. Ceresa, B.P., and S.L. Schmid. 2000. Regulation of signal transduction by endocytosis. *Curr Opin Cell Biol.* 12:204-210.
20. Hobert, M.E., L.A. Friend, and C.R. Carlin. 1999. Regulation of EGF signaling by cell polarity in MDCK kidney epithelial cells. *J Cell Physiol.* 181:330-341.
21. Kuwada, S.K., K.A. Lund, X.F. Li, P. Cliften, K. Amsler, L.K. Opresko, and H.S. Wiley. 1998. Differential signaling and regulation of apical vs. basolateral EGFR in polarized epithelial cells [see comments]. *Am J Physiol.* 275:C1419-1428.
22. Wilson, P.D. 1997. Epithelial cell polarity and disease. *Am J Physiol.* 272:F434-442.
23. Tong, W.M., A. Ellinger, Y. Sheinin, and H.S. Cross. 1998. Epidermal growth factor receptor expression in primary cultured human colorectal carcinoma cells. *Br J Cancer.* 77:1792-1798.
24. Kaech, S.M., C.W. Whitfield, and S.K. Kim. 1998. The LIN-2/LIN-7/LIN-10 complex mediates basolateral membrane localization of the C. elegans EGF receptor LET-23 in vulval epithelial cells. *Cell.* 94:761-771.
25. Whitfield, C.W., C. Benard, T. Barnes, S. Hekimi, and S.K. Kim. 1999. Basolateral localization of the Caenorhabditis elegans epidermal growth factor receptor in epithelial cells by the PDZ protein LIN-10. *Mol Biol Cell.* 10:2087-2100.
26. Pawson, T., and J.D. Scott. 1997. Signaling through scaffold, anchoring, and adaptor proteins. *Science.* 278:2075-2080.
27. Whitmarsh, A.J., and R.J. Davis. 1998. Structural organization of MAP-kinase signaling modules by scaffold proteins in yeast and mammals. *Trends Biochem Sci.* 23:481-485.
28. Burack, W.R., and A.S. Shaw. 2000. Signal transduction: hanging on a scaffold. *Curr Opin Cell Biol.* 12:211-216.
29. Fialka, I., C. Pasquali, R. Kurzbauer, F. Lottspeich, and L.A. Huber. 1999. Loss of epithelial polarity is accompanied by differential association of proteins with intracellular membranes [published erratum appears in Electrophoresis 1999 Apr-May;20(4-5):1122]. *Electrophoresis.* 20:331-343.
30. Fialka, I., C. Pasquali, F. Lottspeich, H. Ahorn, and L.A. Huber. 1997. Subcellul'ar fractionation of polarized epithelial cells and identification of organelle-specific proteins by two-dimensional gel electrophoresis. *Electrophoresis.* 18:2582-2590.
31. Pasquali, C., I. Fialka, and L.A. Huber. 1997. Preparative two-dimensional gel electrophoresis of membrane proteins. *Electrophoresis.* 18:2573-2581.
32. Pasquali, C., I. Fialka, and L.A. Huber. 1999. Subcellular fractionation, electromigration analysis and mapping of organelles. *J Chromatogr B Biomed Sci Appl.* 722:89-102.
33. Fujiki, Y., A.L. Hubbard, S. Fowler, and P.B. Lazarow. 1982. Isolation of intracellular membranes by means of sodium carbonate treatment: application to endoplasmic reticulum. *J Cell Biol.* 93:97-102.
34. Krause, K.H., and M. Michalak. 1997. Calreticulin. *Cell.* 88:439-443.
35. Somsel Rodman, J., and A. Wandinger-Ness. 2000. Rab GTPases coordinate endocytosis. *J Cell Sci.* 113 Pt 2:183-192.
36. Sorkin, A.D., L.V. Teslenko, and N.N. Nikolsky. 1988. The endocytosis of epidermal growth factor in A431 cells: a pH of microenvironment and the dynamics of receptor complex dissociation. *Exp Cell Res.* 175:192-205.
37. Ullrich, O., Reinsch, S., Urbe, S., Zerial, M., and R.G. Parton. 1996. Rab11 regulates recycling through the pericentriolar recycling endosome. *J. Cell Biol.,* 135:913-924.
38. Kobayashi, T., E. Stang, K.S. Fang, P. de Moerloose, R.G. Parton, and J. Gruenberg. 1998. A lipid associated with the antiphospholipid syndrome regulates endosome structure and function [see comments]. *Nature.* 392:193-197.
39. Clague, M.J., S. Urbe, F. Aniento, and J. Gruenberg. 1994. Vacuolar ATPase activity is required for endosomal carrier vesicle formation. *J Biol Chem.* 269:21-24.
40. Schaeffer, H.J., A.D. Catling, S.T. Eblen, L.S. Collier, A. Krauss, and M.J. Weber. 1998. MP1: a MEK binding partner that enhances enzymatic activation of the MAP kinase cascade. *Science.* 281:1668-1671.
41. Choy, E., V.K. Chiu, J. Silletti, M. Feoktistov, T. Morimoto, D. Michaelson, I.E. Ivanov, and M.R. Philips. 1999. Endomembrane trafficking of ras: the CAAX motif targets proteins to the ER and Golgi [see comments]. *Cell.* 98:69-80.
42. Freeman K, Gwadz M, Shore D 1995. Molecular and genetic analysis of the toxic effect of RAP1 overexpression in yeast. Genetics 141(4):1253-62
43. Caumont AB, Jamieson GA, Pichuantes S, Nguyen AT, Litvak S, Dupont C 1996. Expression of functional HIV-1 integrase in the yeast Saccharomyces cerevisiae leads to the emergence of a lethal phenotype: potential use for inhibitor screening. Curr Genet. 29 (6) :503-10
44. Magdolen V, Drubin DG, Mages G, Bandlow W 1993. High levels of profilin suppress the lethality caused by overproduction of actin in yeast cells. FEBS Lett. 316(1):41-7
45. Young K, Lin S, Sun L, Lee E, Modi M, Hellings S, Husbands M, Ozenberger B, Franco R, 1998. Identification of a calcium channel modulator using a high throughput yeast two-hybrid screen. Nat Biotechnol 16(10):946-50
46. Fialka, I., H. Schwarz, E. Reichmann, M. Oft, M. Busslinger, and H. Beug. 1996. The estrogen-dependent c-JunER protein causes a reversible loss of mammary epithelial cell polarity involving a destabilization of adherens junctions. J Cell Biol. 132:1115-1132.
47. Gruenberg, J., and J.P. Gorvel. 1992. In vitro reconstitution of endocytic vesicle fusion. In Protein Targetting, A Practical Approach. A.I. Magee and T. Wileman, editor. Oxford University Press, Oxford. 187-215.
48. Kozak, M. 1999. Initiation of translation in prokaryotes and eukaryotes. Gene. 234:187-208
49. Rubino M, Miaczynska M, Lippe R, Zerial M. 2000. Selective membrane recruitment of EEA1 suggests a role in directional transport of clathrin-coated vesicles to early endosomes. J Biol Chem. 2000 Feb 11;275(6):3745-8
50. Liou, W., H.J. Geuze, M.J. Geelen, and J.W. Slot. 1997. The autophagic and endocytic pathways converge at the nascent autophagic vacuoles. J Cell Biol. 136:61-70.
51. Parton, R.G., M. Way, N. Zorzi, and E. Stang. 1997. Caveolin-3 associates with developing T-tubules during muscle differentiation. J Cell Biol. 136:137-154.
52. Remington's Pharmaceutical Sciences, 1980, Mack Publ. Co., Easton, PA, Osol (ed.).

## Claims

1. A method for identifying compounds for the treatment of disorders mediated by deregulation of the MAPK pathway related to structural and/or functional alterations of the endosomal/lysosomal system, **characterized in that** compounds are tested for their ability to inhibit, on late endosomes/lysosomes, the interaction of p14 with MP1 or the interaction of the p14/MP1 complex with components of the MAPK pathway.

2. The method of claim 1, wherein, in a first step, a cell-free assay is employed to determine the compound's effect on the physical interaction of p14 and MP1.

3. The method of claim 2, wherein the first step comprises
a) immobilizing one of the interaction partners p14 or MP1 on a matrix;
b) labeling the other interaction partner with a detectable marker
c) performing the complex formation between MP1 and p14 or incubating the pre-formed p14/MP1 complex in the presence or absence of the test compound
d) comparing the signal intensity of the treated and the untreated sample.

4. The method of claim 1, wherein, in a first step, a cell-free assay is employed to test the compounds for their ability to interfere with the interaction of the p14/MP1 complex with members of the MAPK pathway.

5. The method of claim 4, wherein the members of the MAPK pathway are selected from MEK1 and/or ERK1 and other proteins that bind to MEK1 or ERK1, either directly or indirectly.

6. The method of any one of claims 1 to 5, wherein the compounds identified in the first step are further tested for their ability to restore disorders related to endosomal/lysosomal phenotypes.
